# EUROPÄISCHE PATENTSCHRIFT

(11) **EP 2 095 843 B1**
(45) Veröffentlichungstag und Bekanntmachung des Hinweises auf die Patenterteilung: **01.12.2010**
(21) Anmeldenummer: 08003840.9
(22) Anmeldetag: 29.02.2008
(51) Int. Cl.: A61N 7/00, A61B 17/22, A61H 1/00, B06B 1/18

(54) **Vorrichtung zur Behandlung biologischer Körpersubstanzen mit mechanischen Druckwellen**
Device for treating biological body substances with mechanical pressure waves
Dispositif de traitement de substances corporelles biologiques à l'aide d'ondes de pression mécaniques

(43) Veröffentlichungstag der Anmeldung: 02.09.2009
(73) Patentinhaber: STORZ MEDICAL AG, 8274 Tägerwilen (CH)
(72) Erfinder: Marlinghaus, Ernest H., Dr., 8598 Bottighofen/TG (CH); Novak, Pavel, Dr., 8234 Stetten (CH); Katona, Josef, 78315 Radolfzell (DE)
(74) Vertreter: Szynka, Dirk

(56) Entgegenhaltungen:
- WO-A1-2008/061645
- DE-A1- 3 342 879
- DE-A1- 3 439 434
- DE-A1- 19 522 403
- DE-A1-102005 022 034
- GB-A- 2 279 290
- US-A- 4 067 549
- US-B1- 6 217 530

## Beschreibung

Diese Erfindung bezieht sich auf eine Vorrichtung.

Solche Vorrichtungen sind an sich bereits bekannt, insbesondere aus dem Bereich der Lithotripsie. Dort werden mit fokussierten mechanischen Druckwellen Körperkonkremente, insbesondere Steine im Körpergewebe, zertrümmert. Neben der Erzeugung durch elektrische Entladungen in Wasser sind auch Geräte entwickelt worden, die mechanische Druckwellen durch das Aufeinanderprallen eines beschleunigten Schlagteils und eines Prallkörpers erzeugen und mit Hilfe des Prallkörpers in Körpergewebe einkoppeln. Solche Geräte sind sowohl in der Lithotripsie mit einem direkten Kontakt zwischen dem Prallkörper bzw. einer mit dem Prallkörper verbundenen Sonde und dem Stein als auch bei anderen Behandlungen von biologischen Körpersubstanzen eingesetzt worden. Insbesondere sind hier die Behandlung von Muskelerkrankungen und von Erkrankungen im Übergangsbereich zwischen Muskeln und Knochen zu nennen.

Ein Beispiel für ein Gerät, das zu dem zusetzt genannten Typ zu rechnen ist, ist die in der EP 0 991 447 dargestellte Vorrichtung. Bei dieser sollen unfokussierte Druckwellen in das Körpergewebe eingekoppelt werden.

Die DE 34 39 434 A1 zeigt eine Vorrichtung zur Erzeugung von mechanischen Schwingungen in Schläuchen, Kathetern, Kanülen oder Nadeln, die in den menschlichen Körper eingeführt sind. Ein schwingend angetriebenes Element stößt gegen ein festes, wobei beide Elemente aus Keramik bestehen können.

Die DE 10 2005 022 034 A1 zeigt den Oberbegriff des Anspruchs 1 und schlägt statt bzw. zusätzlich zu Metall als Werkstoff für einen Prallkörper Kunststoff vor, um die Dämpfungseigenschaften weicher und einstellbar zu gestalten.

Die DE 33 42 879 A1 bezieht sich auf einen Bohrhammer mit keramischen Bauteilen.

Die DE 195 22 403 A1 betrifft ein invasives Gerät zur in vivo Reinigung von Körper kanälen und Gefäßen mit einer fluidangetriebenen Fräseinrichtung aus Keramik.

Die GB 2 279 290 A beschreibt eine Vorrichtung zum Erzeugen einer kreisrunden Öffnung in Keramiken oder Glas durch Kombination einer Longitudinalbewegung einer Spitze im Mikrometerbereich, erzeugt durch ein Piezoantrieb und eine kreisende Bewegung der Spitze in der Ebene senkrecht dazu.

Der Erfindung liegt das technische Problem zugrunde, eine verbesserte Vorrichtung zur Behandlung von biologischen Körpersubstanzen mit einem bewegbaren Schlagteil und einem Prallkörper zur Erzeugung einer mechanischen Druckwelle anzugeben.

Hierzu sind vorgesehen eine Vorrichtung zur Behandlung von biologischen Körpersubstanzen durch Aufsetzen auf einen Patienten mit einem bewegbaren Schlagteil und einem Prallkörper, die dazu ausgelegt ist, durch Beschleunigen des Schlagteils und Aufprallen des Schlagteils auf den Prallkörper eine mechanische Druckwelle in die biologische Körpersubstanz einzukoppeln, **dadurch gekennzeichnet, dass** der Prallkörper aus gesinterter Keramik besteht,
und ein zugehöriges Herstellungsverfahren.
Bevorzugte Ausgestaltungen sind in den abhängigen Ansprüchen angegeben.

Die Erfindung geht von dem Grundgedanken aus, die im Stand der Technik bekannten metallischen Prallkörper, für die insbesondere rostfreier Stahl verwendet wurde, durch ein vorteilhaftes anderes Material, nämlich gesinterte Keramik, zu ersetzen. Keramik zeigt nicht nur eine sehr gute Biokompatibilität und insbesondere geringe Allergierisiken. Es hat sich überraschenderweise herausgestellt, dass gesinterte Keramiken durchaus auch ausreichende Schlagzähigkeit und Härte für den Einsatz als Prallkörper aufweisen und nicht etwa, wie ursprünglich vermutet, unter wiederholter Beaufschlagung mit dem Schlagteil reißen und zerspringen.

Damit ergibt sich die Möglichkeit, je nach Anwendungsfall unterschiedliche vorteilhafte Eigenschaften bestimmter Keramiken einzusetzen. Neben der bereits erwähnten Biokompatibilität betrifft dies auch die regelmäßig im Vergleich zu Metallen deutlich geringere Wärmeleitfähigkeit. Der Patient fühlt jedenfalls bei direkter Berührung der Haut subjektiv ein wärmeres und damit angenehmeres, weniger fremd wirkendes Teil.

Zudem sind viele Keramiken deutlich leichter als übliche Metalle, insbesondere rostfreier Stahl. Dies hat den Vorteil, dass das aus baulichen Gründen üblicherweise kleinere Schlagteil bei einer üblichen metallischen Ausführung einen kleineren Masseunterschied zu dem Prallkörper aufweist und damit die Stoßbedingungen im Sinne eines möglichst großen Impuls- und Energieübergangs verbessert werden. Zudem können auch etwas größere Auslenkungen des Prallkörpers erzielt werden, was von Interesse sein kann.

Schließlich zeigen viele Keramiken auch günstige akustische Impedanzen, die sich von den typischen akustischen Impedanzen von Körpergewebe weniger unterscheiden als Metalle, etwa rostfreier Stahl. Dies ist im Wesentlichen Ergebnis der geringeren Dichte, aber auch von der Schallgeschwindigkeit abhängig.

Bei einzelnen Anwendungen der Erfindung müssen die vorstehenden Vorteile nicht vollständig realisiert werden. Die Erfindung bietet vielmehr die Möglichkeit, je nach Anwendungsfall die eine oder andere positive günstige Eigenschaft von Keramik stärker in den Vordergrund zu rücken.

Unter Keramik wird hier ein aus anorganischen feinkörnigen Rohstoffen gewonnenes Material verstanden, das gesintert ist, also einen Temperschritt erfahren hat. Besonders bevorzugt sind Oxide, insbesondere Metalloxide, Carbide, insbesondere Metallcarbide und Nitride, sowie Mischungen daraus. In Betracht kommen beispielsweise Zirkonoxid oder Siliziumnitrid. Bevorzugt liegt der relative Anteil dieser Materialien bei mindestens 80 Gew.-%, besser 85 Gew.-%, 90 Gew.-% bzw. 95 Gew.-%.

Andere Bestandteile können aber ebenfalls enthalten sein. Insbesondere können in einem gewissen Anteil von maximal 20 Gew.-%, besser maximal 15 Gew.-% bzw. 10 Gew.-% bzw. 5 Gew.-% metallische Anteile vorhanden sein, also metallische Partikel oder Pulver. Aus dem Bereich der Pulvermetallurgie sind für Metalle auch ähnliche Verarbeitungstechniken wie die Keramiksinterung bekannt. Bei den genannten Werten werden die positiven Eigenschaften der Keramik durch diese metallischen Zusätze nicht wesentlich verschlechtert. Im günstigsten Fall sind allerdings keine metallischen Anteile vorhanden.

Eine günstige und bereits bewährte Prallkörpergeometrie ist im Wesentlichen zylindrisch, also zumindest weitgehend rotationssymmetrisch, und weist eine Eintrittsgrenzfläche zur Schlagteilseite und eine Austrittsgrenzfläche zur Körperseite hin auf, die jeweils die Zylinderachse mindestens senkrecht schneiden, vorzugsweise überhaupt senkrecht zur Zylinderachse sind. Sie können allerdings auch leicht gewölbt sein, insbesondere konvex. Der Mantel des Zylinders kann aus verschiedenen Gründen gestuft oder anderweitig mit nicht konstantem Radius gestaltet sein. Insbesondere kann er Strukturen für eine Lagerung mit zumindest einem Elastomerring aufweisen, beispielsweise einen Flansch.

Neben Elastomerringen können auch andere Federn, etwa Schraubenfedern, zur Entkopplung des Prallkörpers von dem Vorrichtungsgehäuse dienen. In Betracht kommen auch wellschlauchähnliche Konstruktionen mit elastischen Eigenschaften.

Eine andere bevorzugte Geometrie des Prallkörpers basiert auf einem um eine zu der Bewegungsrichtung des Schlagteils parallele Längsachse symmetrischen Rotationsellipsoiden, der an der Körpersubstanzseite zu mindestens bis zu dem gewebeseitigen Brennpunkt des Rotationsellipsoiden verkürzt ist und an der Schlagteilseite bis zu dem schlagteilseitigen Brennpunkt verkürzt ist. Bei dieser Ausführungsform sollen fokussierte Druckwellen, die in vergleichsweise einfacher Weise durch das Aufprallen des Schlagteils auf den Prallkörper erzeugt werden, in den Körper eingekoppelt werden. Hierzu soll der Prallkörper als Fokussierelement dienen, um die durch das Aufprallen des Schlagteils in ihm erzeugten Druckwellen zu bündeln. Dies kann erreicht werden durch einen Rotationsellipsoiden als Prallkörper, dessen Längsachse zu der Bewegungsrichtung des Schlagteils parallel sein soll bzw. dieser entsprechen soll.

Zur Erzeugung und Einkopplung der Druckwelle ist der Rotationsellipsoid schlagteilseitig so verkürzt, dass das Schlagteil in dem entsprechenden Brennpunkt auftrifft. Demzufolge bündeln die dem Rötationsellipsoiden entsprechenden Mantelflächenanteile die Druckwelle auf den zweiten Brennpunkt. Der Prallkörper ist an dieser Körpersubstanzseite so verkürzt, dass dieser Brennpunkt entweder unmittelbar auf der an dem Prallkörper anliegenden Körperoberfläche oder davon beabstandet in dem Körper zu liegen kommt. Somit wird durch die Rotationsellipsoidform eine fokussierte Druckwelle in den Körper eingebracht. Durch das Maß der Verkürzung kann eingestellt werden, ob der Brennpunkt auf der Hautoberfläche im Hautbereich oder tiefer im Gewebe liegt, je nachdem auf welchen Körperbereich die Behandlung abzielt.

Der Rotationsellipsoid erlaubt dabei eine kompakte und baulich und in der Handhabung praktische Form des Prallkörpers, die sich leicht montieren lässt, und bietet im Übrigen eine Fokussiermöglichkeit ohne Laufzeitunterschiede zwischen den verschiedenen fokussierten Anteilen. In einer Ellipse werden nämlich die durch einen Brennpunkt gehenden "Strahlen" an der elliptischen Linie so reflektiert, dass sie durch den anderen Brennpunkt laufen, wobei die verschiedenen Varianten zwischen den beiden Brennpunkten identische Weglängen haben.

Vorzugsweise ist der Prallkörper schlagteilseitig und an der dem Körper zugewandten Seite stumpf abgeschnitten.

Verwendet werden pneumatische Antriebe, die mit geringem technischen Aufwand ausreichende Antriebsleistungen und Beschleunigungen für das Schlagteil zur Verfügung stellen. Beispielsweise können damit günstige Geschwindigkeiten des Schlagteils im Bereich von 5 m/s bis 50 m/s erzielt werden. Verwendet wird dabei ein repetitiver Betrieb, und zwar mit Frequenzen zwischen 1 Hz und 50 Hz.

Im Unterschied zu Ausführungen in dem eingangs zitierten Stand der Technik kann es im Rahmen dieser Erfindung bevorzugt sein, mit relativ großen Hüben, d. h. makroskopischen lateralen Verschiebungen des Prallkörpers gegenüber der Gesamtvorrichtung zu arbeiten. Hierbei können insbesondere Werte von mindestens 0,5 mm, besser mindestes 0,6 mm gewählt werden. Es hat sich herausgestellt, dass die für viele Anwendungsfälle wesentliche Druckwellenenergie tatsächlich durch die makroskopische Hubbewegung des Prallkörpers in das zu behandelnde Gewebe eingekoppelt wird und die in dem Prallkörper selbst entstehende und sich ausbreitende akustische Druckwelle (die also nicht einer makroskopischen Schwerpunktsbewegung des Prallkörpers zuzuordnen ist) in vielen Fällen keine wesentliche Rolle spielt. Ein ausreichender Hub sorgt für eine effektive Einkopplung der Druckwellenenergie in diesem Sinn.

Schließlich sind bestimmte Parameter der verwendeten Keramik bevorzugt, insbesondere eine relativ geringe Dichte von vorzugsweise unter 6 g/cm³, besonders bevorzugter Weise unter 5 g/cm³ und noch bevorzugter Weise unter 4 g/cm³. Eine niedrige Dichte verringert die Masse des Prallkörpers und damit die (günstigerweise mit der Hand zu handhabende) Masse des mobilen Teils der Vorrichtung. Sie reduziert auch die bereits erwähnte akustische Impedanz in günstiger Weise. Schließlich ermöglicht sie eine gewisse Baugröße des Prallkörpers ohne zu große Massenunterschiede zwischen Prallkörper und Schlagteil.

Bevorzugt ist ferner eine sog. Schlagzähigkeit des Keramikmaterials von mindestens 3000 kJ/m², besser 4000 kJ/m² und noch günstiger 5000 kJ/m² oder mehr. Diese Größe bestimmt, mit welcher Heftigkeit das Schlagteil auf den Prallkörper auftreffen kann, ohne den Prallkörper selbst zu gefährden.

Schließlich sind relativ harte Materialien bevorzugt, insbesondere solche mit einer größeren Druckfestigkeit von über 2000 MPa.

Keramik bietet schließlich die Möglichkeit, das Material unproblematisch und weitgehend beliebig zu färben. Neben dekorativen Gesichtspunkten kann dies in vorteilhafterweise dazu genutzt werden, unterschiedliche Prallkörpertypen leicht von einander unterscheidbar zu machen. Bei vielen Anwendungsfällen stehen dem Nutzer unterschiedliche Prallkörper zur Verfügung, die im Gerät ausgetauscht werden können. Bei einer farblichen Kodierung sind Fehler unwahrscheinlicher als bei einer alphanumerischen Bezeichnung (die natürlich zusätzlich vorhanden sein kann).

Schließlich ist die Verwendung der Vorrichtung gerade bei der Behandlung von Körperweichgewebe, beispielsweise Muskeln oder Sehnen, bevorzugt. Dies schließt die Behandlung knochennaher Bereiche und eine Stoßwellenakupunktur ein. Typische Indikationen sind Ansatztendinosen und andere Anwendungen in Orthopädie und Chirurgie wie Kalkschultern, Fersenschmerzen, Pseudarthrosen, aber auch Muskelzerrungen. Weitere Indikationen gibt es in der Neurologie, etwa die Verbesserung der Motorik nach Schlaganfällen, die Behandlung von Spasmen nach Traumen sowie Poly-Neuropathien. In der Urologie können etwa chronische Beckenbodenschmerzen behandelt werden; in der Angiologie / Dermatologie und Chirurgie außerdem Narben oder Hautverbrennungen behandelt sowie eine Verbesserung der Wundheilung erzielt werden.

Hinsichtlich der Herstellung des Keramikprallkörpers sind solche Sinterverfahren bevorzugt, bei denen auf den Rohling bzw. werdenden Prallkörper Druck ausgeübt wird. Dies kann vor und/oder während des Temperschritts erfolgen. Insbesondere kann eine isostatische Nachverdichtung unter Hitzeeinwirkung erfolgen.

Im Folgenden wird die Erfindung anhand eines Ausführungsbeispiels näher erläutert, wobei die einzelnen Merkmale auch in anderen als den dargestellten Kombinationen erfindungswesentlich sein können und sich grundsätzlich auf die Vorrichtungs-, die Verwendungs- und die Verfahrenskategorie der Erfindung beziehen.
Figur 1 zeigt einen schematisierten Schnitt entlang der Längsachse durch ein erfindungsgemäßes erstes Ausführungsbeispiel.
Figur 2 zeigt einen analogen Schnitt durch ein zweites Ausführungsbeispiel.

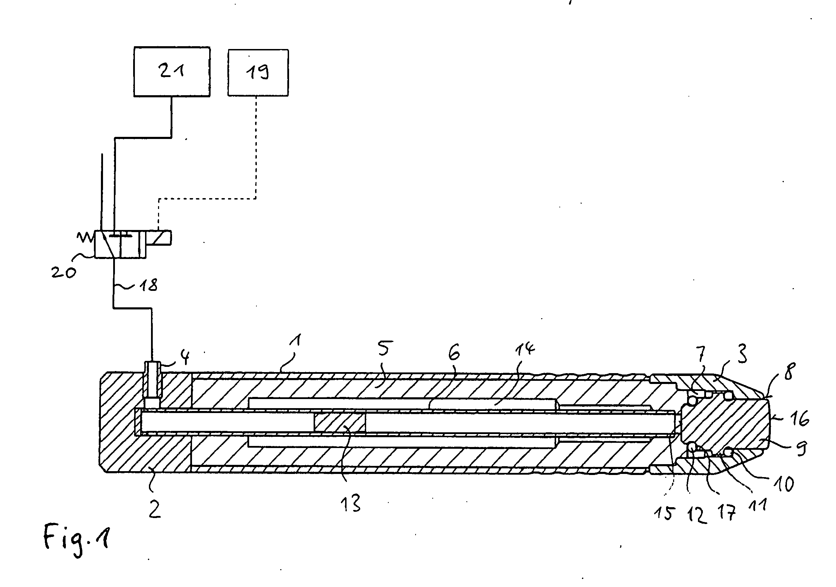

In Figur 1 ist in einem Schnitt entlang einer Längsachse eine erfindungsgemäße Vorrichtung zur Einkopplung von fokussierten mechanischen Druckwellen in beispielsweise den menschlichen Körper dargestellt. Ein Rohrstück bildet ein Gehäuse 1, das von einer in der Anwendung körperfernen Zuluftkappe 2 und einer in der Anwendung körperzugewandten Applikatorkappe 3 jeweils endseitig abgeschlossen ist.

Die Zuluftkappe 2 enthält einen Druckluftanschluss 4 für eine pneumatische Versorgung 18. In an sich bekannter Weise ist an diesen Druckluftanschluss 4 über die pneumatische Versorgungsleitung 18 ein von einer Ansteuereinheit 19 gesteuertes Ventil 20, insbesondere Magnetventil, abgeschlossen, das in einem gleich bleibenden iterativen Takt zwischen etwa 1 Hz und 50 Hz Druckluftpulse über den Druckluftanschluss einkoppelt.

Die Vorrichtung ist als mit der Hand einer Bedienungsperson zu haltendes Gerät ausgebildet, das über die erwähnte Pneumatikleitung 18 an eine Basisstation mit der Ansteuereinheit 19 und dem Kompressor 21 verbunden ist und auf den Patienten manuell aufgesetzt werden kann. Es dient zur Behandlung von Weichgewebe, insbesondere Muskeln.

Die Einzelheiten der Pneumatikversorgung sind nicht Gegenstand dieser Erfindung und dem Fachmann aus dem Stand der Technik geläufig. Vorzugsweise ist die Frequenz einstellbar. Der iterative Betrieb kann komplizierter als mit einer einfachen gleich bleibenden Wiederholung von Pulsen einer bestimmten Frequenz erfolgen, insbesondere auch mit einer Mehrzahl in relativ kurzem Zeitabstand, also mit einer relativ hohen Frequenz, aufeinander folgenden Schlägen, wobei Gruppen von Schlägen in diesem kürzeren Zeitabstand durch etwas größere Zeitabstände voneinander getrennt sind. Die Einzelheiten hierzu sind nicht Gegenstand der vorliegenden Erfindung, können aber damit kombiniert sein.

In dem Gehäuse 1 ist über einen Einsatz 5 ein Führungsrohr 6 gehalten, dessen bei der Anwendung körperfernes Ende in der Zuluftkappe 2 endet und dort mit dem Druckluftanschluss 4 kommuniziert. Das in der Anwendung körperseitige Ende des Führungsrohres 6 endet in einem Teil des Einsatzes 5, der in die Applikatorkappe 3 hineinragt, und zwar kurz vor dem dortigen Ende des Einsatzes 5 und einem Innenraum 7 in der Applikatorkappe 3.

In dem Innenraum 7, der mit zwei radialen Schultern in eine in der Anwendung körperseitige Applikatoröffnung 8 übergeht, ist ein Prallkörper 9 aufgenommen. Dieser stützt sich über einen O-Ring 10 aus einem Elastomer an einer der radialen Schultern ab und weist hierzu einen Flansch 11 auf. Ein zur körperfernen Seite gerichtetes Ende 15 des Prallkörpers 9 stützt sich über einen weiteren O-Ring 12 an dem Einsatz 5 ab, und zwar an einer das bereits erwähnte Ende des Einsatzes 5 umgebenden Stirnfläche. Dabei liegt der O-Ring 12 zwischen dieser Stirnfläche und einem Flansch 17 bzw. einer Schulter des Prallkörpers 9. Die Applikatoröffnung 8 dient dabei zu einer in der Längsrichtung verschiebbaren Führung des Prallkörpers 9 und fixiert diesen quer zur Längsrichtung. Die Axialverschiebbarkeit ist durch die Nachgiebigkeit der Elastomerringe 10 und 12 begrenzt und liegt bei in Luft betriebener Vorrichtung relativ zur Restvorrichtung deutlich über 0,6 mm.

Auf die Merkmale des Prallkörpers 9, der hier gleichzeitig den auf die Haut aufzusetzenden Applikator bildet, wird im Folgenden noch näher eingegangen.

In dem angrenzenden Bereich des Führungsrohres 6 ist ein in Figur 1 mit dem Prallkörper 9 in Kontakt stehendes Schlagteil 13 eingesetzt. Dieses passt (in Bezug auf das Führungsrohr und die im Wesentlichen zylindrische Geometrie des Schlagteils 13) radial mit geringem Spiel. Das Schlagteil 13 kann durch Druckunterschiede der Luftsäule in dem Führungsrohr 6 vor und hinter ihm (d. h. in Figur 1 rechts und links des Schlagteils 13) in dem Führungsrohr hin- und herbewegt werden und insbesondere auf den Prallkörper 9 zu beschleunigt werden. Hierzu wird es aus einer Ausgangsposition (nicht gezeigt) in Figur 1 links durch einen Druckluftstoß durch den Druckluftanschluss 4 beschleunigt und trifft mit seiner dem Prallkörper 9 zugewandten Frontfläche (in Figur 1 der Übersichtlichkeit halber nicht bezeichnet) auf den Prallkörper 9 auf.

Die Rückbewegung des Schlagteils 13 erfolgt durch ein Rückströmen der Luft aus einer das Führungsrohr 6 innerhalb des Einsatzes 5 umgebenden Staukammer 14. In diese wird die Luft bei der Beschleunigung des Schlagteils 13 in Richtung zu dem Prallkörper 9 verdrängt und damit dort komprimiert. Wenn das Magnetventil 20 in der Pneumatikzuleitung 18 des Druckluftanschlusses 4 den Druck wegschaltet, wird das Schlagteil 13 damit in die Ausgangsstellung zurückbewegt. Dies kann natürlich auch durch eine zusätzliche oder alternative Druckbeaufschlagung der Staukammer 14 oder eines anderen Luftvolumens körperseitig von dem Schlagteil 13 erfolgen. Das in der Anwendung körperfeme Ende des Führungsrohres 6 endet in einem Magnethalter 17 für das Schlagteil 13.

Der Prallkörper 9 hat eine rotationssymmetrische Zylinderform und ist in axialer Richtung durch die Eintrittsfläche 15 und die etwas konvexe Austrittsfläche 16 begrenzt. Der Außenmantel weist die bereits beschriebenen flanschartigen Strukturen 11 und 17 auf, die Anlageschultem für die O-Ringe 10 und 12 bilden. Im Übrigen ist ein austrittsseitiger Teil der Zylindergeometrie mit konstantem Radius gestaltet und damit in der Öffnung 8 axial verschiebbar.

Erfindungsgemäß besteht der Prallkörper 9 aus gesinterter Keramik, und zwar einem verdichtetem Siliciumnitrid-Material (Si₃N₄). Es handelt sich um polykristallines Material mit tetragonaler Kristallstruktur, das sich als erstaunlich schlagzäh und fest erwiesen hat. Quantitativ kann die Schlagzähigkeit angegeben werden mit 6500 - 7000 kJ/m² bei einer Druckfestigkeit von etwa 3000 MPa.

Das Material ist relativ leicht, verfügt nämlich über eine Dichte von 3,2 g/cm³. Da die Schallgeschwindigkeit für longitudinale Druckwellen zwar höher als in rostfreiem Stahl liegt, aber nicht zu hoch, ergibt sich eine um etwa 20 % - 25 % reduzierte akustische Impedanz, die dementsprechend näher an der akustischen Impedanz vom Körpergewebe liegt. Die Einkopplung der Druckwelle in das Körpergewebe erfolgt damit noch etwas günstiger als bei konventionellen Prallkörpern.

Das Material hat ferner eine Wärmeleitfähigkeit in der Größenordnung von 20 W/mK und wirkt damit sensorisch weniger kalt als Stahl. Es ist auf Biokompatibilität getestet.

Hier kann beispielhaft verwiesen werden auf biologische Versuche, über die berichtet wird in "Biokompatibilität von Siliziumnitrid-Keramik in der Zellkultur. Eine vergleichende fluoreszenzmikroskopische und rasterelektronenmikroskopische Untersuchung", Laryngo-Rhino-Otol 2004, 83: 845-851, auch in Thieme-connect des Georg Thieme Verlags und Thieme Medical Publishers, Inc.

Schließlich kann der Prallkörper 9 unproblematisch gefärbt werden (etwa durch Zusatz von farbigen Metallionen wie Co). sodass unterschiedlich geformte und/oder unterschiedlich schwere Prallkörper zur Variation verschiedener Behandlungsparameter, insbesondere des Hubes, der Größe der Austrittsfläche 16 oder ihrer Form usw. durch verschiedene Farben gekennzeichnet und verwechslungssicher montiert werden können. Dazu ist die Applikatorkappe einfach abschraubbar.

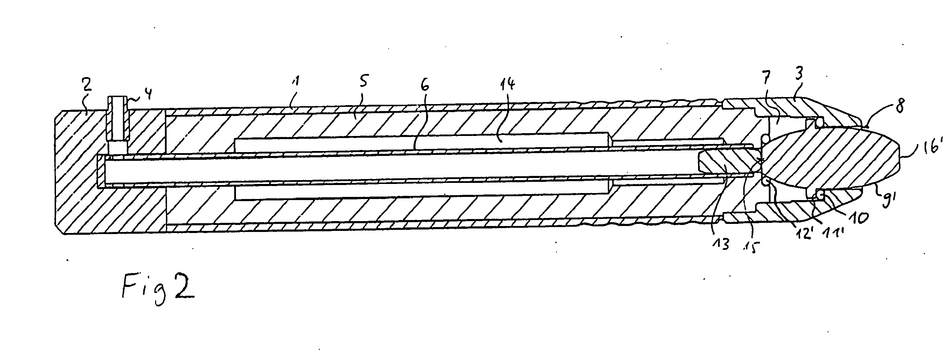

In Figur 2 ist ein analoger Schnitt durch ein zweites Ausführungsbeispiel zur Einkopplung von fokussierten mechanischen Druckwellen dargestellt. Es sind weitgehend die gleichen Bezugszeichen wie in Figur 1 eingetragen, die nicht erneut erläutert werden. Abweichend ausgeführt ist lediglich die Form des Prallkörpers 9' als Rotationsellipsoid.

Dieser stützt sich über einen O-Ring 10 aus einem Elastomer an einer der radialen Schultern ab und weist hierzu einen Flansch 11' auf. Ein sich zur körperfernen Seite hin verjüngendes Ende des Prallkörpers 9' stützt sich über einen weiteren O-Ring 12' an dem Einsatz 5 ab, und zwar in einem ein Loch in dem bereits erwähnten Ende des Einsatzes 5 umgebenden Einschnitt.

Das linke und rechte (ursprünglich) konvexeste Ende des rotationselliptischen Prallkörpers 9' jeweils symmetrisch zur Längsachse ist jeweils gerade abgeschnitten. Dabei läuft die linke Schnittfläche 15' als zu der Längsachse senkrechte plane Fläche durch den dortigen Brennpunkt des Ellipsoiden bzw. der im Schnitt dargestellten Ellipse. Das Gleiche gilt für die rechte Schnittfläche 16' mit der Maßgabe, dass dort die Schnittfläche durch den zweiten Brennpunkt oder weiter innen läuft und hier abgerundete Kanten aufweist.

Die Schnittfläche 16' könnte auch innerhalb des zweiten Brennpunktes verlaufen, sodass der zweite Brennpunkt unter der Hautoberfläche im Körpergewebe liegt, was in vielen Fällen bevorzugt ist.

Die Schlagteilfrontfläche kann auch etwas konvex sein, um eine eher punktförmige Welleneinkopplung zu erreichen. Ferner kann der Hub deutlich kleiner als 0,5 mm bemessen werden, weil hier eher der Anteil der durch den Prallkörper 9' laufenden fokussierten Druckwelle von Interesse ist.

## Patentansprüche

1. Vorrichtung zur Behandlung von biologischen Körpersubstanzen eines Patienten durch Aufsetzen auf den Patienten mit
- einem bewegbaren Schlagteil (13) und
- einem Prallkörper (9, 9'),
die dazu ausgelegt ist, durch Beschleunigen des Schlagteils (13) und Aufprallen des Schlagteils (13) auf den Prallkörper (9, 9') eine mechanische Druckwelle in die biologische Körpersubstanz einzukoppeln,
**dadurch gekennzeichnet, dass** der Prallkörper (9, 9') aus gesinterter Keramik mit einer Dichte von höchstens 6 g/cm³ besteht,
und durch einen pneumatischen Antrieb zur Beschleunigung des Schlagteils (13) mit einer Frequenz zwischen 1 Hz und 50 Hz.

2. Vorrichtung nach Anspruch 1, bei der die Keramik zu mindestens 80 Gew.-% aus Oxiden, Carbiden und/oder Nitriden besteht.

3. Vorrichtung nach Anspruch 1 oder 2, bei der der Prallkörper (9) eine zylindrische Form mit einer die Zylinderachse senkrecht schneidenden schlagteilseitigen Eintrittsfläche (15) und einer die Zylinderachse ebenfalls senkrecht schneidenden körpersubstanzseitigen Austrittsfläche (16) aufweist.

4. Vorrichtung nach Anspruch 1 oder 2, bei der der Prallkörper (9') einen Mantelflächenanteil aufweist, der einen Teil eines um eine zu der Bewegungsrichtung des Schlagteils (13) parallele Längsachse symmetrischen Rotationsellipsoiden bildet,
wobei der Prallkörper (9') an der Körpersubstanzseite (16') mindestens bis zu dem gewebeseitigen Brennpunkt des Rotationsellipsoiden verkürzt ist und
an der Schlagteilseite (15') bis zu dem schlagteilseitigen Brennpunkt verkürzt ist.

5. Vorrichtung nach einem der vorstehenden Ansprüche, bei der der Prallkörper (9, 9') über mindestens einen, vorzugsweise zwei Elastomerringe (10, 12, 12')
in der Vorrichtung gehalten und dabei gegen den elastischen Widerstand des/der Elastomerringe/s axial verschiebbar in der Vorrichtung geführt ist.

6. Vorrichtung nach Anspruch 5, bei der der Prallkörper (9, 9') mindestens einen Flansch (11,11') zur Abstützung gegen die Elastomerringe (10, 12, 12') aufweist.

7. Vorrichtung nach einem der vorstehenden Ansprüche, bei der die Keramik eine Schlagzähigkeit von mindestens 3 MPam aufweist.

8. Vorrichtung nach einem der vorstehenden Ansprüche, bei der die Keramik eine Druckfestigkeit von mindestens 2000 MPa aufweist.

9. Vorrichtung nach einem der vorstehenden Ansprüche mit einer Mehrzahl unterschiedlicher Prallkörper (9, 9'), die im Austausch gegeneinander montierbar und durch unterschiedliche Färbung unterscheidbar sind.

10. Vorrichtung nach einem der vorstehenden Ansprüche, die ausgelegt ist zur Behandlung und insbesondere zur Stoßwellenakupunktur von Körperweichgewebe, insbesondere Muskeln oder Sehnen, und knochennaher Bereiche; beispielsweise zur Behandlung von Ansatztendinosen und anderen Anwendungen in Orthopädie und Chirurgie wie Kalkschultern, Fersenschmerzen, Pseudarthrosen, aber auch Muskelzerrungen, in der Neurologie, etwa zur Verbesserung der Motorik nach Schlaganfällen, Behandlung von Spasmen nach Traumen sowie Poly-Neuropathien, in der Urologie, etwa zur Behandlung von chronischen Beckenbodenschmerzen, in der Angiologie/Dermatologie und in der Chirurgie, etwa zur Behandlung von Narben oder Hautverbrennungen sowie zur Verbesserung der Wundheilung.

11. Verfahren zu Herstellung einer Vorrichtung nach einem der Ansprüche 1 - 10, bei der der Keramikprallkörper (9, 9') gepresst und thermisch behandelt wird.

## Claims

1. An apparatus for treating biological body substances of a patient by positioning said apparatus against said patient, said apparatus having
- a movable projectile (13) and
- an impact body (9, 9'),
and being designed for coupling a mechanical shockwave into said biological body substance by accelerating said projectile (13) and colliding said projectile (13) with said impact body (9, 9'),
**characterized in that** said impact body (9, 9') consists of a sintered ceramic with a density of 6 g/cm³ at maximum,
and by a pneumatic drive for accelerating said projectile (13) with a frequency between 1 Hz and 50 Hz.

2. The apparatus of claim 1, wherein at least 80 percent in weight of said ceramic consist of oxides, carbides and/or nitrides.

3. The apparatus according to claim 1 or 2, wherein said impact body (9) has a cylindrical shape with a projectile-facing entry face (15) intersecting the cylinder axis perpendicularly and a body substance-facing exit face (16) intersecting said cylinder axis also perpendicularly.

4. The apparatus according to claim 1 or 2, wherein said impact body (9') has a lateral area part forming a part of an ellipsoid of revolution, which is symmetrical to a longitudinal axis being parallel to the moving direction of said projectile (13),
wherein said impact body (9') is shortened at least up to the tissue-facing focal point of said ellipsoid of revolution at the body substance face (16) and is shortened up to the projectile-facing focal point at its projectile face (15').

5. The apparatus according to one of the preceding claims, wherein said impact body (9, 9') is hold by at least one, preferably two, elastomer rings (10, 12, 12') in said apparatus, being guided therein axially movable against the elastic resistance of said elastomer ring/s in said apparatus.

6. The apparatus according to claim 5, wherein said impact body (9, 9') comprises at least one flange (11, 11') for a support against said elastomer rings (10, 12, 12').

7. The apparatus according to one of the preceding claims, wherein said ceramic has an impact resistance of at least 3 MPam.

8. The apparatus according to one of the preceding claims, wherein said ceramic has a compression resistance of at least 2000 Mpa

9. The apparatus according to one of the preceding claims with a plurality of different impact bodies (9, 9'), which can be mounted exchanging each other and are distinguishable due to a different colouring.

10. The apparatus according to one of the preceding claims being designed for a treatment, in particular for a shockwave acupuncture, of soft body tissue, in particular muscles or tendons, and of regions close to the bones, for example for treating insertion tendinitis and for other applications in orthopedics and surgery, as calcareous tendinitis of the shoulder, heel pain, pseudo-arthrosis, but also muscle sprains, in neurology, for example for improving the motor function after a stroke, treatment of spasm after traumas as well as poly-neuropathies, in urology, for example for treating a chronic pain of the pelvic floor, in angiology/dermatology and in surgery, for example for treating scars or a skin burning as well as for improving the wound healing.

11. A method for manufacturing an apparatus according to one of claims 1 - 10, wherein said ceramic impact body (9, 9') is compacted and thermally treated.

## Revendications

1. Dispositif de traitement de substances corporels biologiques d'un patient par pose sur le patient comprenant :
- une pièce de percussion mobile (13) et
- un corps de rebond (9, 9'),
qui est conçu pour envoyer une onde de choc mécanique sur la substance corporelle biologique par l'accélération de la pièce de percussion (13) et la percussion de la pièce de percussion (13) sur le corps de rebond (9, 9'),
**caractérisé en ce que** le corps de rebond (9, 9') est en céramique frittée d'une masse volumique maximale de 6g/cm³.
et par un entraînement pneumatique pour accélérer la pièce de percussion (13) ayant une fréquence comprise entre 1 Hz et 50 Hz.

2. Dispositif selon la revendication 1, dans lequel la céramique est composée au moins à 80 % en poids d'oxydes, de carbures et/ou de nitrures.

3. Dispositif selon la revendication 1 ou 2, dans lequel le corps de rebond (9) présente une forme cylindrique avec une face d'entrée (15) côté pièce de percussion coupant verticalement l'axe du cylindre et une face de sortie (16) côté substance corporelle coupant également verticalement l'axe du cylindre.

4. Dispositif selon la revendication 1 ou 2, dans lequel le corps de rebond (9') présente une partie d'enveloppe qui forme une partie d'une ellipsoïde de rotation symétrique sur un axe longitudinal parallèle au sens de déplacement de la pièce de percussion (13),
sachant que le corps de rebond (9') est réduit sur le côté de substance corporelle (16') au moins jusqu'au centre côté tissu de l'ellipsoïde de rotation et est réduit sur le côté pièce de percussion (15') jusqu'au centre côté partie de percussion.

5. Dispositif selon l'une des revendications précédentes, dans lequel le corps de rebond (9, 9') est maintenu dans le dispositif par au moins une, de préférence deux bagues élastomère (10, 12, 12') et peut ainsi être poussé guidée axialement dans le dispositif contre la résistance élastique de la/des bague(s) élastomère.

6. Dispositif selon la revendication 5, dans lequel le corps de rebond (9, 9') présente au moins une bride (11, 11') pour s'appuyer contre les bagues élastomère (10. 12, 12').

7. Dispositif selon l'une des revendications précédentes, dans lequel la céramique présente une résilience d'au moins 3 MPam.

8. Dispositif selon l'une des revendications précédentes, dans lequel la céramique présente une résistance à la pression d'au moins 2000 MPam.

9. Dispositif selon l'une des revendications précédentes comprenant une pluralité de corps de rebond (9, 9') différents qui peuvent être montés échangés l'un contre l'autre et peuvent être différenciés par des couleurs différentes.

10. Dispositif selon l'une des revendications précédentes conçu pour le traitement et en particulier pour l'acupuncture par ondes de choc de tissus mous corporels, en particulier de muscles ou de tendons, et de zones proches des os : par exemple pour le traitement d'enthésites et d'autres applications en orthopédie et chirurgie comme les calcifications de l'épaule, les douleurs du talon, les pseudarthroses mais aussi les froissements musculaires, en neurologie pour améliorer par exemple la motricité après des accidents vasculaires-cérébraux, le traitement des spasmes après des traumatismes et les polyneuropathies, en urologie par exemple pour le traitement des douleurs chroniques de la ceinture pelvienne, en angiologie/dermatologie et en chirurgie par exemple pour traiter les cicatrices ou les brûlures de la peau et améliorer la cicatrisation des plaies.

11. Procédé de fabrication d'un dispositif selon l'une des revendications 1 à 10 dans lequel le corps de rebond en céramique (9, 9') est comprimé et subit un traitement thermique.
